# EUROPEAN PATENT SPECIFICATION

(11) **EP 3 981 367 B1**
(45) Date of publication and mention of the grant of the patent: **04.11.2026**
(21) Application number: 21203729.5
(22) Date of filing: 20.06.2014
(51) Int. Cl.: A61F 2/89, A61F 2/915, A61F 2/04

(54) **STENT WITH DEFLECTING CONNECTOR**
STENT MIT ABLENKENDEM VERBINDER
ENDOPROTHÈSE À CONNECTEUR DE DÉFLEXION

(30) Priority: 21.06.2013 US 201361837770 P
(43) Date of publication of application: 13.04.2022
(62) Document number(s) of the earlier application(s) in accordance with Art. 76 EPC: 14738997.7 / 3 010 451
(73) Proprietor: Boston Scientific Scimed Inc., Maple Grove, Minnesota 55311 (US)
(72) Inventor: SEDON, Dane T, Boston, Massachusetts 02127 (US); ROSS, Daniel, Watertown, Minnesota 55388 (US); DORAN, Burns P., Monticello, Minnesota 55362 (US); FLEURY, Sean P, Brighton, Massachusetts 02135 (US); WOOD, Mark D., Shrewsbury, Massachusetts 01545 (US)
(74) Representative: Vossius & Partner Patentanwälte Rechtsanwälte mbB

(56) References cited:
- WO-A1-2009/103011
- WO-A2-2007/079363
- DE-A1- 10 150 547
- US-A1- 2005 222 671
- US-A1- 2006 015 173
- US-A1- 2006 036 312
- US-A1- 2010 137 973

## Description

### BACKGROUND

A stent is a medical device introduced into a body lumen. A stent is typically delivered in an unexpanded state to a desired location in a bodily lumen and then expanded by an internal radial force. Stents, grafts, stent-grafts, vena cava filters, expandable frameworks, and similar implantable medical devices, collectively referred to hereinafter as stents, are radially expandable endoprostheses, which are typically intravascular implants capable of being implanted transluminally.

Stents have previously been introduced into the trachea in order to address a variety of medical issues: to provide additional support to the trachea itself and/or the surrounding tissue following surgery, to prevent the airway from being constricted from tumor in growth, to alleviate stenosis, etc.

Tracheal stents face a unique environment of use, one in which the deployed stent must expand and contract during respiration and also be capable of providing support to the trachea.

When referring to tracheal stents, removability and flexibility are often the two things physicians speak about when referring to a great stent. Removability allows the physician the option to place a stent with confidence in treatable malignant conditions, as well as benign conditions, without the dangers of leaving an implant behind. Flexibility of a stent translates to comfort for a patient, e.g., a stent that does not force the lumen in to a straightened path offers reduced irritation. This disclosure will describe stents having geometries which exhibit both of these properties.

US 2006/036312 A1 discloses a stent that has a folded strut section that provides both structural rigidity and reduction in foreshortening of the stent mechanism. A flexible section provides flexibility for delivery of the stent mechanism. In a second device variant, flexible section columns are angled with respect to each other, and to the longitudinal axis of the stent. These relatively flexible sections are oppositely phased in order to negate any torsion along their length.

US 2005/222671 A1 discloses a stent which after expansion within the body remains substantially rigid for a certain time, at which point the stent becomes increasingly conformable, due to the biodegradation of the connecting members, and thereby avoids mechanical irritation to the body's tissue.

US 2010/137973 A1 discloses an implantable medical device having an outer stent with a tubular body disposed about a longitudinal axis.

WO 2007/079363 A2 discloses a helical stent having bioresorbable connecting members connecting sections of the stent.

US 2006/015173 A1 discloses a stent that includes at least one annular element defined by a first set of strut members interconnected to define apices proximate opposite sides of the annular element. The annular element further includes a foot extension extending between at least one pair of circumferentially-adjacent strut members.

WO 2009/103011 A1 discloses a prosthesis that comprises a tubular body that is expandable from a contracted configuration to a radially expanded configuration. The tubular body has a total length and comprises a first section, a second section and a central section disposed therebetween. The total length of the tubular body in the expanded configuration is at least 95% of the total length of the tubular body in the contracted configuration. The three sections have a plurality of tubular rings, each with a plurality of struts having a length and coupled together to form a series of peaks and valleys. A connector couples adjacent tubular rings together. The length of the central section struts is different than the length of the other struts and the central section is coupled with both the first and second sections.

DE 101 50 547 A1 discloses a stent comprising at least two annular-segments formed of sloping struts continuously adjoining each other in a meander shape by means of curved sections. Adjacent annular segments are connected by connectingstruts. The curved sections between two sloping struts are staggered in position.

### SUMMARY

The present invention is defined by the features of claim 1. Preferred embodiments are defined by the features of the dependent claims, respectively. As mentioned above, embodiments of the present disclosure are directed to stents and stent geometries which provide improved flexibility and removability characteristics. Some embodiments are directed to stents for use in a mammalian trachea.

As mentioned above, embodiments of the stent disclosed herein are provided with geometries that provide the stent with desired tracheal flexiblility as well as allow the stent to be readily removed from the trachea following either short-term or long term deployment.

Removability: The stent geometry is designed in such a way that it allows axial extension and compression to mimic the anatomical environment's extreme conditions. The connectors of the embodiments disclosed herein are configured to be in tension with one another and provide minimal diameter shift, direct pull force translation, and increased durability. These features are improvements over known stents in that prior stents are known to fracture and pull apart if there is significant tissue in-growth anchoring the stent to the anatomy; this is due to the stent cells distorting beyond the designed intent and inducing high stress regions. In the arrangements of the stent connectors shown and described herein, the stress concentration is in straightening the offset connector allowing for greater force to be displaced without creating fracture.

Flexibility: Often times in existing stents the tradeoff between flexibility and removability leaves one of these attributes with diminished performance. To achieve the requisite level of flexibility, the stent should have stent geometry that allows for inside and outside chord length changes. In order for this to happen, the cell design is usually weakened allowing the distortion or deflection to come from a shift in the cell geometry. In embodiments disclosed herein, the stent connectors are provided with an offset design, which lends itself to allowing these distortions to be displaced directly without significantly affecting the cell geometry. This provides a multitude of advantages: it allows the radial and indenter force to maintain consistency throughout a deflection, keeps indenter force high while allowing for a great deal of flexibility, prevents kinking/ovaling during deflection, and it also maintains the ability of the stent to be removed.

Embodiments of the stent described herein have the ability to axially extend or compress at least 20% or more of the stent's nominal deployed length without significantly altering the deployed diameter of the stent or suffering permanent deformation. In some embodiments, the stents described herein have the ability to axially extend or compress up to 40% or more of the nominal deployed length without significantly altering the deployed diameter of the stent or suffering permanent deformation.

**In** some embodiments, a tracheal stent comprises an expandable tubular member having a proximal end, a distal end, a longitudinal axis extending through the proximal and distal ends, an inner surface, and an outer surface. The stent comprises a plurality of strut columns and at least one connector extending between each strut column. The ends of the at least one connector are longitudinally and circumferentially offset from one another. **In** some embodiments the at least one connector extends from a peak of a strut pair of one strut column to a trough of a strut pair in a circumferentially adjacent strut column.

**In** some embodiments, the at least one connector comprises a first axial segment extending from a first end of a circumferential segment and second axial segment extending from a second end of the circumferential segment. **In** some embodiments the tracheal stent has a nominal state and an axially extended state. **In at** least one embodiment the tracheal stent has an axially shortened or compressed state.

**In** the nominal state the first axial segment and the circumferential segment define a nominal angle of about 90 degrees to about 115 degrees. **In** the axially extended state the first axial segment and the circumferential segment define an angle greater than that of the nominal angle. **In** the axially shortened state the first axial segment and the circumferential segment define an angle less than that of the nominal angle.

**In** at least one embodiment, in the axially extended state the first axial segment and the circumferential segment define an angle about 125 degrees to about 180 degrees.

**In** the nominal state the second axial segment and the circumferential segment define a nominal angle of about 90 degrees to about 115 degrees. **In** the axially extended state the second axial segment and the circumferential segment define an angle greater than that of the nominal angle. **In** the axially shortened state the second axial segment and the circumferential segment define an angle less than that of the nominal angle.

**In** at least one embodiment, in the axially extended state the second axial segment and the circumferential segment define an angle about 125 degrees to about 180 degrees.

**In** the various embodiments described herein a tracheal stent has a length. **In** the axially extended state the length is at least 20% greater than the length of the stent in the nominal state. **In** some embodiments when the stent is in the axially extended state the length is up to 40% greater than the length of the stent in the nominal state.

### BRIEF DESCRIPTION OF THE DRAWINGS

FIG. 1 is a flat view of a portion of a stent in a nominal state, according to one embodiment;
FIG. 2 is a flat view of the stent portion shown in FIG. 1 in an axially extended state, according to one embodiment;
FIG. 3 is an annotated close-up view of a connector and adjacent strut columns of the stent shown in FIG. 1, wherein the annotations depict examples of configurations of the connector in an axially compressed state and an axially extended state, according to one embodiment;
FIG. 4 illustrates the entire stent depicted in FIG. 1 in a laboratory setting with the stent in an axially extended state, according to one embodiment; and
FIG. 5 is a flat view of a portion of another embodiment of the stent.

### DETAILED DESCRIPTION

For the purposes of this disclosure, like reference numerals in the figures shall refer to like features unless otherwise indicated.

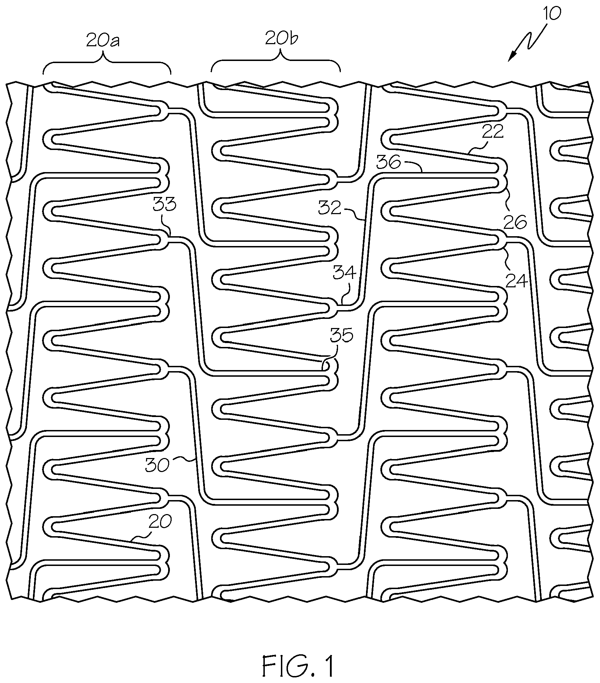

A partial view of a stent 10 is depicted in FIG. 1. In the embodiment shown, the stent 10 is depicted in a nominal deployed state. In an environment of use the nominal deployed or extended state of the stent 10 is a state wherein the outer surface of the stent is in contact with the trachea (not shown) of a patient, and the trachea is at rest (between inspiration and expiration events). Alternatively, the nominal deployed state can be defined as a state wherein the stent 10 is expanded to its programmed shape-memory deployed diameter.

As is shown in the various figures 1-5, the stent 10 is comprised of a plurality of strut columns 20. Each strut column 20 is comprised of a series of interconnected struts 22 which form alternating peaks 24 and troughs 26. Adjacent strut columns 20 are connected by one or more connectors 30.

Each connector 30 is comprised of a circumferential segment 32 and first and second axial segments 34 and 36, which extend in substantially opposite directions from the opposing ends of the circumferential segment 32.

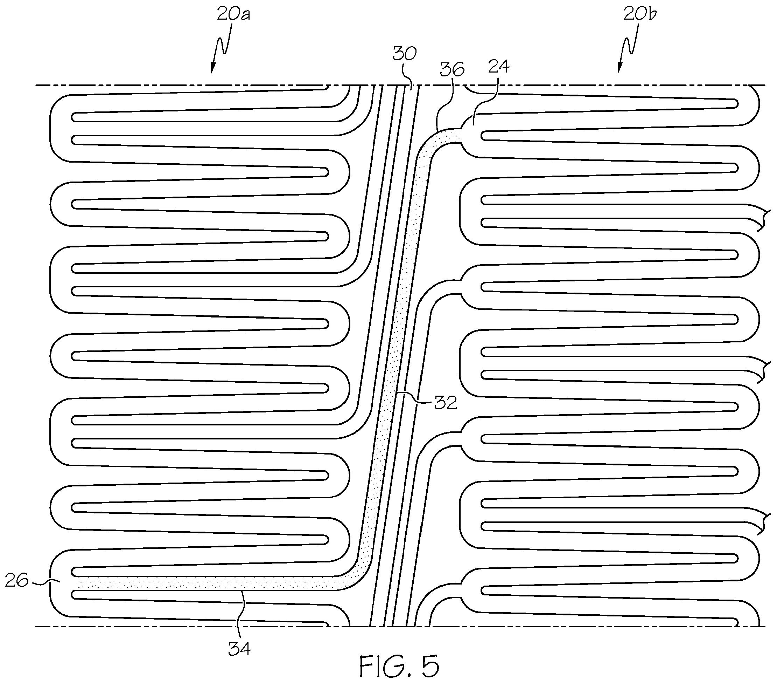

In the embodiments shown in FIG. 1-4, a first (or proximal) end 33 of each connector 30 extends from a peak 24 of a first (or proximal) strut column 20a. A second (or distal) end 35 of each connector 30 extends from a trough 26 of a second (or distal) strut column 20b. In some embodiments the connectors 30 can extend from a trough 26 of the first strut column 20a and engage a peak 24 of the second strut column 20b such as in the manner depicted in FIG. 5.

In the various embodiments described herein, the length of the circumferential segment 32 results in the first and second axial segments 34 and 36 being circumferentially offset from one another in the nominal state. The length of the circumferential segment 32 is such that it extends in a circumferential direction across at least one trough 26 of the first strut column 20a and at least one peak 24 of the second strut column 20b. The length of the circumferential segment 32 can vary a great deal. For example, in the alternative embodiment shown in FIG. 5, the length of the circumferential segment 32 is sufficient to cross over six peaks 24 of the first strut column 20a and five troughs 26 of the second strut column 20b. Various other lengths and configurations of peak and trough crossings can be provided to the connectors 30.

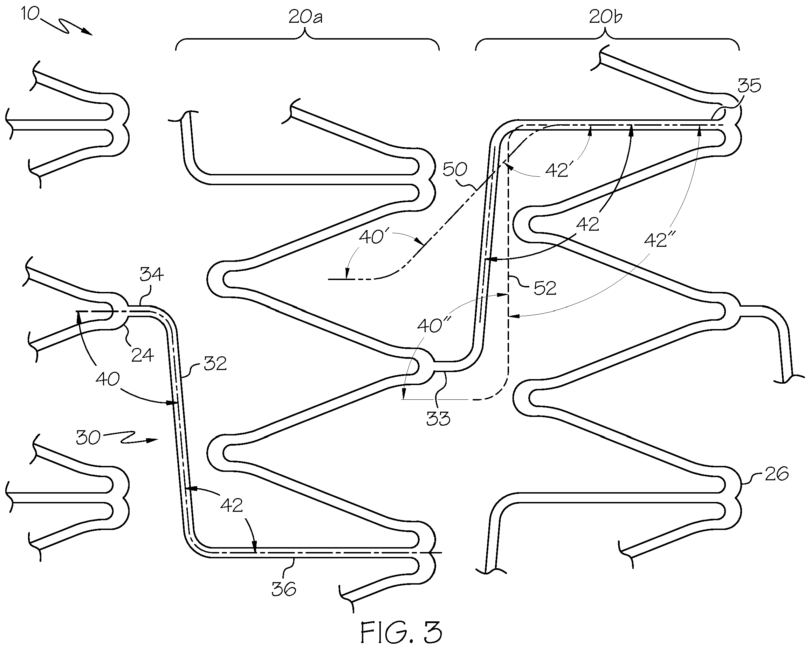

In the nominally extended state shown the first axial segment 34 and the circumferential segment 32 define a nominal angle 40 of about 90 degrees to about 115 degrees, as shown in FIG. 3. Similarly, the second axial segment 36 and the circumferential segment 32 define a nominal angle 42 of about 90 degrees to about 115 degrees. In some embodiments angles 40 and 42 define alternate interior angles.

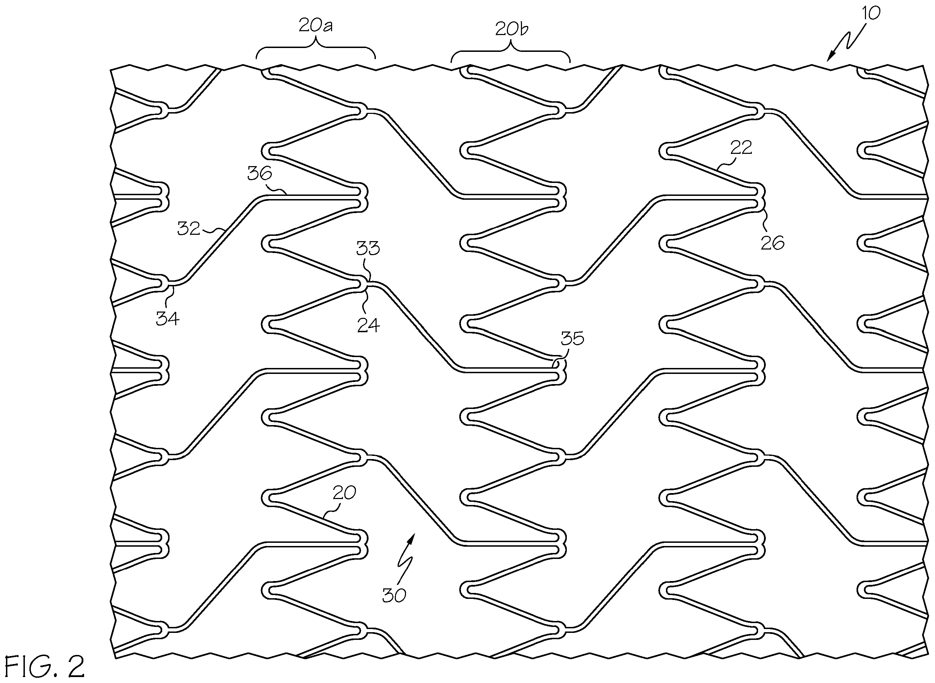

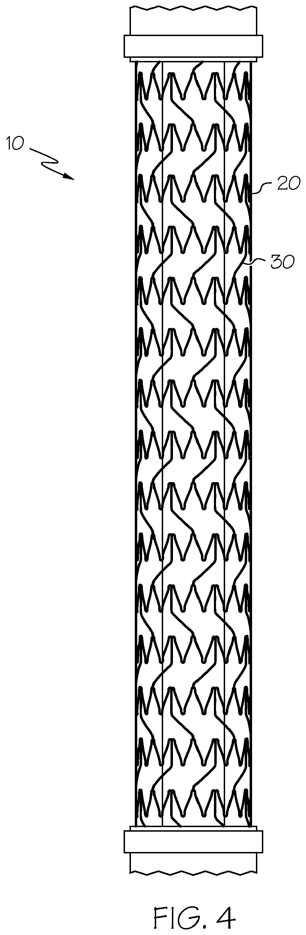

When deployed within the trachea, stent 10 is configured to be capable of extending from the nominally deployed state shown in FIG. 1 to an axially extended state, such as is shown in FIGs. 2 and 4. In the axially extended state the stent may have an axial length 20 percent greater (or more) than the length of the stent 10 in the nominally deployed state. As illustrated via FIG. 2, when the stent 10 is expanded to the axially extended state, deflection of the connectors 30 permits the stent 10 to axially elongate such that a majority of the axial elongation results from deflection of the connectors 30 rather than from distortions of the strut columns 20.

In the axially extended state the first axial segment 34 and the circumferential segment 32 define an angle 40' greater than that of the nominal angle 40. In at least one embodiment, in the axially extended state the first axial segment 34 and the circumferential segment 32 define an angle 40' of about 125 degrees to about 180 degrees. Likewise, in the axially extended state the second axial segment 36 and the circumferential segment 32 define an angle 42' greater than that of the nominal angle 42. In at least one embodiment, in the axially extended state the second axial segment 36 and the circumferential segment 32 define an angle 42' of about 125 degrees to about 180 degrees. In some embodiments angles 40' and 42' define alternate interior angles.

In addition to being capable of extending axially during an inspiration event, embodiments of the stent 10 are also configured to adapt to expiration events wherein the trachea may compress in the axial direction. An example of the extent to which a connector 30 can "extend" from the nominally deployed state to an axially extended state is illustrated by annotation line 50 and an example of the extent to which the connector 30 can compress from the nominally deployed state to an axially compressed state is shown by annotation line 52.

When the stent 10 is extended from the nominally deployed state (trachea at rest) to the axially extended state (inspiration), the connectors 30 (as represented by annotation line 50 in FIG. 3) will deflect such that the adjacent strut columns can move axially apart from one another (e.g. axially extend) without significantly affecting the deployed diameter of the stent 10. When the stent 10 is axially compressed (expiration) from the nominally deployed diameter (trachea at rest) in the axially compressed state, the connectors 30 (as represented by annotation line 52 in FIG. 3) will deflect such that the adjacent strut columns can move axially toward one another (e.g. axially compress) without significantly affecting the deployed diameter of the stent 10.

In the various embodiments shown and described herein, when the stent 10 is in the axially compressed or shortened state (as represented by annotation line 52 in FIG. 3), the first axial segment 34 and the circumferential segment 32 define an angle 40" that is less than that of the nominal angle 40. Similarly, in the axially compressed state the second axial segment 36 and the circumferential segment 32 define an angle 42" that is less than that of the nominal angle 42. In some embodiments angles 40" and 42" define alternate interior angles.

The unique geometry of the stent 10 provides the stent 10 with the capability to axially extend or compress by at least 20% or more of its nominal deployed length without significantly altering the deployed diameter of the stent or causing the stent to suffer permanent deformation. In some embodiments, the stent 10 is capable of axially extending or compressing by up to 40% of the nominal deployed length without significantly altering the deployed diameter of the stent or causing the stent to suffer permanent deformation.

In some embodiments, a force required to change the length of the stent from a nominal length to an axially extended length is less than about 2.22 N (0.5 lbs). In at least one embodiment, the force required to change the length of the stent from a nominal length to an axially extended length is about 2.10 N (0.472 lbs). In some embodiments, the force required to change the length of the stent from a nominal length to an axially extended length ranges from about 0.89 N to about 1.11 N (0.2 lbs to 0.25 lbs).

In addition to the above it is recognized that any embodiments of the present stent 10 may be provided with a uniform diameter, may taper in portions or along the entire length of the stent, may have struts 20 and/or connectors 30 with uniform or different widths and/or thicknesses.

Embodiments of stent 10 may be manufactured using any appropriate stent manufacturing techniques. Appropriate methods for manufacturing the stents may include laser cutting, chemical etching or stamping of a tube. The stents may also be manufactured by laser cutting, chemically etching, stamping a flat sheet, rolling the sheet and welding the sheet, by electrode discharge machining, or by molding the stent with the desired design.

Any appropriate stent material may be used in the manufacture of the inventive stent 10. Examples of such materials may include polymeric materials, metals, ceramics and composites. Appropriate polymeric materials include thermotropic liquid crystal polymers (LCP's). Where the stent 10 is made of metal, the metal may be stainless steel, cobalt chrome alloys such as elgiloy, tantalum or other plastically deformable metals. Other suitable metals include shape-memory metals such as nickel-titanium alloys generically known as "nitinol", platinum/tungsten alloys and titanium alloys, stainless steel, tantalum and elgiloy. This disclosure also contemplates the use of more than one material in the manufacture of the stent 10. For example, first strut columns 20a and second strut columns 20b may be made of different materials. Optionally, the connectors 30 may be made of a different material than the strut columns 20.

Embodiments of the stent 10 are self-expanding. However in some embodiments the stent 10 may be provided in mechanically expandable form, in self- or as a hybrid of self-expanding and mechanically expandable. Mechanically expandable stents, in accordance with the disclosure, may be expanded using any suitable mechanical device.

Embodiments of the stent 10 may include suitable radiopaque coatings. For example, the stents may be coated with gold or other noble metals or sputtered with tantalum or other metals. The stents may also be made directly from a radiopaque material to obviate the need for a radiopaque coating or may be made of a material having a radiopaque inner core. Other radiopaque metals which may be used include platinum, platinum-tungsten, palladium, platinum-iridium, rhodium, tantalum, or alloys or composites of these metals.

Embodiments of the stent 10 may be provided with various biocompatible coatings to enhance various properties of the stent. For example, the stents may be provided with lubricious coatings. The stents may also be provided with drug-containing coatings which release drugs over time.

Embodiments of the stent 10 may also be used as the framework for a graft, sleeve, covering or coating (partially or over the entire surface of the stent). Suitable coverings include but are not limited to, nylon, collagen, PTFE and expanded PTFE, polyethylene terephthalate and KEVLAR. More generally, any known graft material may be used including natural or synthetic polymers such as silicone, polyethylene, polypropylene, polyurethane (or urethane), polyglycolic acid, polyesters, polyamides, their mixtures, blends, copolymers, mixtures, blends and copolymers.

## Claims

1. A stent (10) having a nominally deployed state having a deployed diameter, an axially extended state, and an axially compressed state, the stent (10) having a length, in the axially extended state the length being at least 20% greater than in the nominally deployed state;
wherein the stent (10) is configured to axially extend from the nominally deployed state to the axially extended state without significantly altering the deployed diameter of the stent (10);
wherein the stent (10) comprises:
a plurality of strut columns (20), wherein the plurality of strut columns (20) comprise columnar struts interconnected by alternating peaks (24) and troughs (26), and
connector columns, the connector columns comprising connector struts (30) extending between adjacent strut columns (20a, 20b);
wherein the connector struts (30) comprise a first axial segment (34), a second axial segment (36), and a circumferential segment (32), the first axial segment (34) and the second axial segment (36) extending from the circumferential segment (32), the circumferential segment (32) disposed between the first and second axial segments (34, 36);
wherein the connector struts (30) are configured to extend from the nominally deployed state to the axially extended state such that:
in the axially extended state, the first axial segment (34) and the circumferential segment (32) define an angle (40') greater than that of a nominal angle (40) between the first axial segment (34) and the circumferential segment (32) in the nominally deployed state, wherein the nominal angle (40) is in a range of about 90 degrees to about 115 degrees, and
the second axial segment (36) and the circumferential segment (32) define an angle (42') greater than that of a nominal angle (42) between the second axial segment (36) and the circumferential segment (32) in the nominally deployed state, wherein the nominal angle (42) is in a range of about 90 degrees to about 115 degrees.

2. The stent of claim 1, wherein the length of the stent (10) in the axially compressed state is at least 20% less than in the nominally deployed state.

3. The stent of claim 1 or 2, wherein the stent (10) is configured to axially compress from the nominally deployed state to the axially compressed state without significantly altering the deployed diameter of the stent (10).

4. The stent of any one of the preceding claims, wherein the stent (10) is configured to axially extending or compressing up to 40% of its length in the nominally deployed state without significantly altering the deployed diameter of the stent (10).

5. The stent of any one of the preceding claims, wherein a force necessary to change the length of the stent (10) in the nominally deployed state to the axially extended state is less than 4.4 N (0.5 lbs).

6. The stent of any one of the preceding claims, wherein the connector struts (30) extend between adjacent strut columns (20a, 20b) in a peak-to-trough configuration.

7. The stent of claim 6, wherein the first axial segment (34) and the second axial segment (36) are circumferentially offset from each other in the nominally deployed state.

8. The stent of claim 7, wherein the plurality of strut columns (20) comprise a first strut column (20a) and a second strut column (20b), and wherein the circumferential segment (32) extends in a circumferential direction across at least one trough (26) of the first strut column (20a) and at least one peak (24) of the second strut column (20b).

9. The stent of claim 8, wherein the circumferential segment (32) extends in a circumferential direction across at least one trough (26) of the first strut column (20a) and at least two peaks (24) of the second strut column (20b).

10. The stent of any one of claims 1-9, wherein the stent (10) is self-expanding.

11. The stent of any one of claims 1-9, wherein the stent (10) is balloon-expandable.

12. The stent of any one of the preceding claims, further comprising a covering extending over an entire surface of the stent (10).

13. The stent of claim 12, wherein the covering is a silicone covering.

14. The stent of claim 12, wherein the covering is a polyurethane covering.

## Patentansprüche

1. Stent (10) mit einem nominell entfalteten Zustand, der einen entfalteten Durchmesser aufweist, einem axial ausgedehnten Zustand und einem axial komprimierten Zustand, wobei der Stent (10) eine Länge aufweist, wobei die Länge im axial ausgedehnten Zustand mindestens 20% größer ist als im nominell entfalteten Zustand,
wobei der Stent (10) dafür konfiguriert ist, sich vom nominell entfalteten Zustand axial in den axial ausgedehnten Zustand auszudehnen, ohne den entfalteten Durchmesser des Stents (10) wesentlich zu verändern,
wobei der Stent (10) aufweist:
mehrere Strebensäulen (20), wobei die mehreren Strebensäulen (20) Säulenstreben aufweisen, die durch abwechselnde Spitzen (24) und Mulden (26) miteinander verbunden sind; und
Verbindersäulen, wobei die Verbindersäulen Verbindungsstreben (30) aufweisen, die sich zwischen benachbarten Strebensäulen (20a, 20b) erstrecken,
wobei die Verbinderstreben (30) ein erstes axiales Segment (34), ein zweites axiales Segment (36) und ein Umfangssegment (32) aufweisen, wobei sich das erste axiale Segment (34) und das zweite axiale Segment (36) vom Umfangssegment (32) erstrecken und das Umfangssegment (32) zwischen dem ersten und dem zweiten axialen Segment (34, 36) angeordnet ist,
wobei die Verbinderstreben (30) derart konfiguriert sind, dass sie sich vom nominell entfalteten Zustand in den axial ausgedehnten Zustand erstrecken, so dass:
im axial ausgedehnten Zustand das erste axiale Segment (34) und das Umfangssegment (32) einen Winkel (40') definieren, der größer ist als ein nomineller Winkel (40) zwischen dem ersten axialen Segment (34) und dem Umfangssegment (32) im nominell entfalteten Zustand, wobei der nominelle Winkel (40) in einem Bereich von etwa 90 Grad bis etwa 115 Grad ist, und
das zweite axiale Segment (36) und das Umfangssegment (32) einen Winkel (42') definieren, der größer ist als ein nomineller Winkel (42) zwischen dem zweiten axialen Segment (36) und dem Umfangssegment (32) im nominell entfalteten Zustand, wobei der nominelle Winkel (42) in einem Bereich von etwa 90 Grad bis etwa 115 Grad ist.

2. Stent nach Anspruch 1, wobei die Länge des Stents (10) im axial komprimierten Zustand um mindestens 20% geringer ist als im nominell entfalteten Zustand.

3. Stent nach Anspruch 1 oder 2, wobei der Stent (10) dafür konfiguriert ist, sich vom nominell entfalteten Zustand in den axial komprimierten Zustand axial zusammenzuziehen, ohne den entfalteten Durchmesser des Stents (10) wesentlich zu verändern.

4. Stent nach einem der vorangehenden Ansprüche, wobei der Stent (10) dafür konfiguriert ist, sich im nominell entfalteten Zustand um bis zu 40% seiner Länge axial auszudehnen oder zusammenzuziehen, ohne den entfalteten Durchmesser des Stents (10) wesentlich zu verändern.

5. Stent nach einem der vorangehenden Ansprüche, wobei eine Kraft, die erforderlich ist, um die Länge des Stents (10) im nominell entfalteten Zustand in den axial ausgedehnten Zustand zu verändern, weniger als 4,4 N (0,5 lbs) beträgt.

6. Stent nach einem der vorangehenden Ansprüche, wobei sich die Verbinderstreben (30) zwischen benachbarten Strebensäulen (20a, 20b) in einer Spitze-zu-Mulde-Konfiguration erstrecken.

7. Stent nach Anspruch 6, wobei das erste axiale Segment (34) und das zweite axiale Segment (36) im nominell entfalteten Zustand in der Umfangsrichtung voneinander versetzt sind.

8. Stent nach Anspruch 7, wobei die mehreren Strebensäulen (20) eine erste Strebensäule (20a) und eine zweite Strebensäule (20b) aufweisen, und wobei sich das Umfangssegment (32) in der Umfangsrichtung über mindestens eine Mulde (26) der ersten Strebensäule (20a) und mindestens eine Spitze (24) der zweiten Strebensäule (20b) erstreckt.

9. Stent nach Anspruch 8, wobei sich das Umfangssegment (32) in der Umfangsrichtung über mindestens eine Mulde (26) der ersten Strebensäule (20a) und mindestens zwei Spitzen (24) der zweiten Strebensäule (20b) erstreckt.

10. Stent nach einem der Ansprüche 1 bis 9, wobei der Stent (10) selbstexpandierend ist.

11. Stent nach einem der Ansprüche 1 bis 9, wobei der Stent (10) ballonexpandierbar ist.

12. Stent nach einem der vorangehenden Ansprüche, zusätzlich aufweisend eine sich über die gesamte Oberfläche des Stents (10) erstreckende Beschichtung.

13. Stent nach Anspruch 12, wobei die Beschichtung eine Silikonbeschichtung ist.

14. Stent nach Anspruch 12, wobei die Beschichtung eine Polyurethanbeschichtung ist.

## Revendications

1. Stent (10) ayant un état nominalement déployé ayant un diamètre déployé, un état axialement étendu et un état axialement comprimé, le stent (10) ayant une longueur, à l'état axialement étendu, la longueur étant au moins 20% supérieure à celle dans l'état nominalement déployé ;
dans lequel le stent (10) est configuré pour s'étendre, de manière axiale, de l'état nominalement déployé à l'état axialement étendu, sans modifier, de manière significative, le diamètre déployé du stent (10) ;
dans lequel le stent (10) comprend :
une pluralité de colonnes d'entretoises (20), dans lequel la pluralité de colonnes d'entretoises (20) comprend des entretoises colonnaires interconnectées par des pics (24) et des dépressions (26) alternés, et
des colonnes de connecteurs, les colonnes de connecteurs comprenant des entretoises de connecteurs (30) qui s'étendent entre les colonnes d'entretoises (20a, 20b) adjacentes ;
dans lequel les entretoises de connecteurs (30) comprennent un premier segment axial (34), un deuxième segment axial (36) et un segment circonférentiel (32), le premier segment axial (34) et le deuxième segment axial (36) s'étendant à partir du segment circonférentiel (32), le segment circonférentiel (32) étant disposé entre les premier et deuxième segments axiaux (34, 36) ;
dans lequel les entretoises de connecteurs (30) sont configurées pour s'étendre de l'état nominalement déployé à l'état axialement étendu, de sorte que :
à l'état axialement étendu, le premier segment axial (34) et le segment circonférentiel (32) définissent un angle (40') supérieur à un angle nominal (40) entre le premier segment axial (34) et le segment circonférentiel (32) à l'état nominalement déployé, dans lequel l'angle nominal (40) est dans une plage d'environ 90 degrés à environ 115 degrés, et
le deuxième segment axial (36) et le segment circonférentiel (32) définissent un angle (42') supérieur à un angle nominal (42) entre le deuxième segment axial (36) et le segment circonférentiel (32) à l'état nominalement déployé, dans lequel l'angle nominal (42) est dans une plage d'environ 90 degrés à environ 115 degrés.

2. Stent selon la revendication 1, dans lequel la longueur du stent (10), à l'état axialement comprimé, est d'au moins 20% inférieure à celle à l'état nominalement déployé.

3. Stent selon la revendication 1 ou 2, dans lequel le stent (10) est configuré pour appliquer une compression, de manière axiale, de l'état nominalement déployé à l'état axialement comprimé, sans modifier, de manière significative, le diamètre déployé du stent (10).

4. Stent selon l'une quelconque des revendications précédentes, dans lequel le stent (10) est configuré pour s'étendre ou se comprimer de manière axiale jusqu'à 40% de sa longueur à l'état nominalement déployé sans modifier, de manière significative, le diamètre déployé du stent (10).

5. Stent selon l'une quelconque des revendications précédentes, dans lequel une force nécessaire pour modifier la longueur du stent (10) de l'état nominalement déployé à l'état axialement étendu est inférieure à 4,4 N (0,5 lbs).

6. Stent selon l'une quelconque des revendications précédentes, dans lequel les entretoises de connecteurs (30) s'étendent entre les colonnes d'entretoises (20a, 20b) adjacentes dans une configuration pic - dépression.

7. Stent selon la revendication 6, dans lequel le premier segment axial (34) et le deuxième segment axial (36) sont décalés, de manière circonférentielle, l'un de l'autre à l'état nominalement déployé.

8. Stent selon la revendication 7, dans lequel la pluralité de colonnes d'entretoises (20) comprend une première colonne d'entretoises (20a) et une deuxième colonne d'entretoises (20b), et dans lequel le segment circonférentiel (32) s'étend dans une direction circonférentielle sur au moins une dépression (26) de la première colonne d'entretoises (20a) et au moins un pic (24) de la deuxième colonne d'entretoises (20b).

9. Stent selon la revendication 8, dans lequel le segment circonférentiel (32) s'étend dans une direction circonférentielle sur au moins une dépression (26) de la première colonne d'entretoises (20a) et au moins deux pics (24) de la deuxième colonne d'entretoises (20b).

10. Stent selon l'une quelconque des revendications 1 à 9, dans lequel le stent (10) est auto-expansible.

11. Stent selon l'une quelconque des revendications 1 à 9, dans lequel le stent (10) est expansible sur ballonnet.

12. Stent selon l'une quelconque des revendications précédentes, comprenant en outre un revêtement s'étendant sur toute la surface du stent (10).

13. Stent selon la revendication 12, dans lequel le revêtement est un revêtement en silicone.

14. Stent selon la revendication 12, dans lequel le revêtement est un revêtement en polyuréthane.
